# EUROPEAN PATENT APPLICATION

(11) **EP 3 517 142 A1**
(43) Date of publication of application: **31.07.2019**
(21) Application number: 19162289.3
(22) Date of filing: 16.10.2015
(51) Int. Cl.: A61L 15/32, A61L 15/44, A61L 15/46, A61L 15/28, A61L 15/58

(54) **DEVICE FOR WOUND DRESSING**

(30) Priority: 17.10.2014 EP 14189451
(62) Divisional of application: 15798194.5
(71) Applicant: Leonardino S.r.l., 20021 Bollate (MI) (IT)
(72) Inventor: LORENZONI, Stefano, I-20157 Milano MI (IT)
(74) Representative: Gislon, Gabriele

(57) **Abstract**

A wound dressing device comprising at least one layer (2) of silk fibroin nanofibers and an absorbent component (4); the layer of fibroin nanofibers contacts the wound of the user and helps in the healing process.

## Description

The present invention relates to a device for wound dressing. In greater detail the present invention relates to a device for wound dressing comprising nanofibers of silk fibroin.

Several types of devices for wound dressing are known in the art. Simple dressings such as plasters are used to dress simple wounds such as small cuts or bruises; some wounds such as those deriving from burns or diabetes ulcers are chronic, more complicated to treat and often require special wound dressings. The same applies to other wounds such as decubitus or pressure ulcers, dehiscent surgical wounds, i.e. wounds that do not heal in a standard time notwithstanding the use of stitches, and wounds where a non neglectable part of skin and tissue has been lost. In these cases the normal physiological healing process is often stuck and the wound does not cicatrize. For these wounds it is known in the art to provide special wound dressings that could contain drugs or substances that help the cicatrization and the healing of the wound.

It is known to use silk fibroin as a substance for the preparation of wound dressings. Silk fibroin is a protein polymer widely known for its applications as a biomaterial. Derived from *Bombix mori* cocoons, silk fibroin has a series of interesting properties. These properties could be highly controlled through different processing techniques, in order to achieve controllable degradation rates, specific surface properties, high mechanical strength, porosity and gas permeability. It is also possible that fibroin absorbs specific substances, e.g. drugs, growth factors, etc. Finally it can be processed with different methods in order to obtain particular mechanical properties., resulting into different forms such as hydrogels, films, electrospun fibers, sponge-like structures, etc.. In the following description the term fibroin will be used to indicate silk fibroin.

Patent application WO2012040310A2 uses silk fibroin as an optional component of an electrospun fiber comprising activated platelet-rich plasma (aPRP). Such fibers are highly permeable and are ideal for cell adhesion, thanks to the presence of aPRP which makes the fibrous construct similar to a blood product. The presence of aPRP also makes this construct capable of long-term and sustained release of the growth factors present in the aPRP. Thus, this type of fiber is dedicated to use in wound treatment or tissue engineering.

Patent application EP2545943 uses silk fibroin as a possible component of a perforated biomatrix layer. The patent connects at least two such layers with a bonding, adhesive agent. There are at least two perforated biomatrix layers connected by a bonding agent, and more layers can be added in the same manner in order to increase the material's thickness. This multi-layered construct is aimed at sore treatment.

Patent application CN103446617A uses silk fibroin as a way of delivering a gentamicin sulphate/gelatin microsphere complex. The fibroin is used under the form of a scaffold, and is prepared by adding such complex to the silk fibroin solution that will then be used to prepare the silk fibroin scaffold, which will thereby be already loaded with the microsphere complex. This construct has long-acting antibacterial and drug slow-release function, and can be applied to wound healing and dressing.

All these methods either require adding a substance or a coating to the silk fibroin structure, use the silk fibroin as a delivery medium to treat wounds, and/or use the silk fibroin's structural properties to enhance cellular growth. All these modifications are made by the addition of the substance to the fibroin structure after forming said structure; alternatively, the substance and the fibroin are treated together while the silk fibroin is being formed into its preferred form, e.g. microfibers, and thus such a fibroin offers a limited range of applications depending on the agents it carries.

The known medical devices for wound dressing also have problems deriving from the presence of the drugs used in the dressing as a medicament. As an example, the presence of growth factors as the above cited aPRP, could results in the proliferation of bacteria in the wound and in an increased danger of infection. To solve this problem it is known to add to the dressing device antibiotics or a biocidal substance; in a case, Ag is used as a biocide. This solution, however, entails the further problem of jeopardizing the growth of the tissue or to result, as in the case of use of biocidal Ag, into a build-up and accumulation of the biocide in the body of the treated subject.

There is, therefore, a need for a wound dressing device that can be widely applied to many different types of wounds without the need of carrying specific substances in order to be effective, but still capable of being coupled with a specific substance if required.

It is an aim of the invention to solve the above problems and to provide a wound dressing device that can be used with many different types of wounds to support the healing process and to help in fighting off an infection or in avoiding it, in particular from external environment, without the need of carrying specific substances in order to be effective, but still capable of being coupled with a specific substance if required.

It is a further aim of the invention to provide a medical device that can be used in a wide variety of wounds, mostly chronic wounds; in particular, it is an aim of the invention to provide a wound dressing that can be used together with complex wound treatments, e.g. in general any drug or substance which fights off infections and promotes the healing process of the wound, such as topical growth factors, platelet gel, acid or basic substances used as disinfectants, iodoform and any therapies, such as negative pressure wound therapy, and hyperbaric oxygen therapy.

The above mentioned aims are reached by means of the present invention that provides a wound dressing according to claim 1. The wound dressing of the invention comprises a fibroin layer made of nanofibers and further comprises an absorbent component arranged in a multilayer structure. In the use, the fibroin will be facing the wound and the absorbent component will be separated from the wound by the fibroin layer. The absorbent layer is coupled to the nanostructured fibroin layer to maintain a moist environment which promote interactions between the silk fibroin nanofibers and the bacteria. Exemplary interactions may be physical, e.g. electrostatic forces, chemical and/or morphological, e.g. in view of the morphological affinity of the bacteria with the fibroin nanostructure.

The term "nanofiber" herein means that the fibers have an average fiber diameter of less than 900 nm, preferably from 200 to 800 nm.

In the present application the word "wound" is intended to designate any type of alteration or injury of the skin, including contaminated, infected and colonized wounds where the wound is a chronic one and is very difficult to heal, as in a bedsore, ulcer or post-surgical wounds. A wound according to the present invention also covers skin injury such as burn or sunburn wounds, fungal infections.

The fibroin structure is made with nanofibers having an average diameter within the range of 200 to 800 nm, preferably 400 to 700 nm (Measured on a scanning electron microscope SEM-FEG MIRA3; calculated as the arithmetic mean based on 15 samples; preferably with a software using scale bar calibration). Preferably, the fibroin layer is an electrospun fibroin, of the type that has a structure suitable to be used for cell culture in vitro. The structure is preferably made with non-oriented fibers of fibroin but aligned fibers may also be used.

The fibroin layer has a weight in the range of 10-40 g/m² preferably 15-35 g/m² advantageously 25-25 g/m², for instance about 27-29 g/m².

In a most preferred embodiment, the fibroin layer is made of 100% fibroin, which means that it is free from any other component, especially free from PEO (polyethylene oxide) - which is never used during the pre-processing and the processing of the silk cocoon,, drugs, active ingredients and/or biocidal substances that may be released into the body of the user.

Preferably, the absorbent component comprises at least one of viscose, polyester, cellulose, cotton fibers, foams, gel forming particles, gel forming fibers, gauze and wound dressing parts with liquid absorbing properties. The absorbent layer is obtained through traditional technologies, known per se in the art, e.g by a process of obtaining a woven or a non-woven layer. According to a preferred embodiment, the absorbent layer is a mixture of viscose/polyester in a weight ratio 70/30.

In an embodiment of the invention, the absorbent layer is positioned between two layers of fibroin. Such a device may be obtained by simply folding a device made of one fibroin layer and one absorbent layer.

According to a preferred embodiment, the weight ratio between the two layers, i.e. fibroin weight/absorbent weight is in the range 1:10 to 3:4, preferably the ratio is 1:3, i.e. 1/4 fibroin and 3/4 absorbent.

According to another aspect of the invention, the absorbent component may comprise a biocidal substance, i.e. a drug, a medicament, or a similar device.

In a further embodiment, the wound dressing of the invention comprises means to adhere at least part of said dressing, i.e. the fibroin layer(s) coupled to the absorbent layer or absorbent component, to the skin of a user. To this purpose, a further layer is provided, external to the device with respect to the skin of the user, including an adhesive substance suitable to maintain the device on the skin of the user; the dimensions of the external layer with adhesive are selected according to the wound extension. In a further aspect of the invention, the combination of fibroin layer and absorbent component is wide enough to cover the wound and the adjacent healthy skin, so as to avoid that the exudate of the wound pour out onto the healthy skin and possibly infect it.

The fibroin structure is made of nanofibers having an average fiber diameter of less than 900 nm, preferably within the range of 200 to 800 nm, most preferably 400 to 700 nm (Measured with a software using scale bar calibration on a scanning electron microscope SEM-FEG MIRA3). The fibroin layer is preferably obtained by electrospinning a fibroin solution in a way known per se in the art, having an average thickness in a range from a few microns to 200 microns, for instance from 50 to 200 microns. A chemical treatment of crystallization (e.g. with rinses of methanol) is made on the electrospun material to modify the protein structure and to obtain chemical-physical properties of the silk fibroin.

A full disclosure of how to prepare a fibroin layer suitable for the present invention can be found in Eng. Life Sci. 2008, 8, No. 3, 219-225*,* Alessandrino et al. Electrospun Silk Fibroin Mats for Tissue Engineering*,* in particular point 2.1 and table 1. The resulting layer of fibroin nanofibers is suitable for cell growth, i.e. cell proliferation.

Another process to prepare a suitable layer of fibroin is similar to the above mentioned one, but does not make any use of LiBr, instead makes use of formic acid and CaCl₂.

The preferred range of the weight of the fibroin layer is 10-40 g/m², preferably 15-35 g/m² (grams per square meter).

The invention provides several advantages over the prior art. The device is capable of covering a wound while physically removing bacteria from it, without the need of additional substances, e.g. bactericides.

In fact, it was surprisingly found that fibroin nanofibers according to the invention, namely electrospun nanofibers of silk fibroin, operate as elements for the entrapping and removal of bacteria from a wound, in a device for treating wounds. The nanostructured layer of fibroin is morphologically similar to the ECM (extracellular matrix), giving thus a compatible environment in terms of sizes and geometry with most of bacteria, and promoting their adhesion on the said layer. Moreover, the absorbent layer help this binding by creating a moist environment and promoting electrostatic and physical interactions between silk fibroin nanofibers and bacteria.

The wound dressing device comprises a matrix (or layer) composed of electrospun silk fibroin, and a layer of absorbing material that can consist of cotton fibers, cellulose, polyester, or viscose. The silk fibroin will come in contact with the patient, and will separate the patient from the absorbing material while allowing passage of fluids of the exudate in the wound. The fibroin matrix can be either as a single sheet between the wound and the absorbing material, or as two sheets placed on the two sides of the absorbing material.

The silk fibroin matrix has an average diameter between 200 and 800 nm, preferably 400 to 700 nm, and has a density in a range of 0,2-0,4 kg/dm³ preferably 0,25-0,35 Kg/dm³

The absorbing material causes the movement of fluids from the wound through the silk fibroin matrix to the absorbing material itself, which keeps the fibroin layer humid and has it act as a sort of filter. This, coupled with the fibroin's chemical-physical properties, enable the fibroin layer to bind with bacteria carried by the fluid. Bacteria remain bound to the fibroin because of physic and electrostatic forces, e.g. hydrophobic interaction, which are enhanced by the water-rich ambient generated by the absorbing material. In particular, flagellate bacteria tend to tightly bind with the silk fibroin since the flagella have similar dimensions to that of the electrospun silk fibroin nanofibers. Also, the structure of such fibroin layer makes it easy for bacteria to come in contact, and thus interact with the fibroin itself, since the gaps in the structure are in the same order of magnitude of the bacteria and other microorganisms in general.

A further object of the invention is thus the use of fibroin as above disclosed, as an element for entrapping bacteria and is especially useful in a device for treating wounds.

Such device does not need addition of substances to exert its functions but, if desired, such substances can be added as needed, preferably to the absorbent component, since the material does not interfere with eventual therapies that are taking place to heal the wound. As previously mentioned, the invention of this wound dressing device can also be applied in cases of ulcers, post-surgical wounds, burn or sunburn wounds, fungal infections, or in items such as tampons and diapers.

The invention will now be further disclosed with reference to the following exemplary and non-limiting drawings, wherein:
- Figure 1 is a schematic cross sectional view of one embodiment of the wound dressing according to the invention;
- Figure 2A and 2B are schematic cross sectional views of a further embodiment of the wound dressing according to the invention;
- Figures 3A and 3B are schematic cross sectional views of variants of the embodiments of Figures 1 and 2 respectively;
- Figures 4-5 are SEM images of details of the layer of fibroin nanofibers,
- Figures 6-7-8 are SEM images of details and cross sectional view of the device of the invention.

It should be noticed that the Figures are not showing the real-life dimensions of the device and of its components with respect to a wound and with respect to each other.

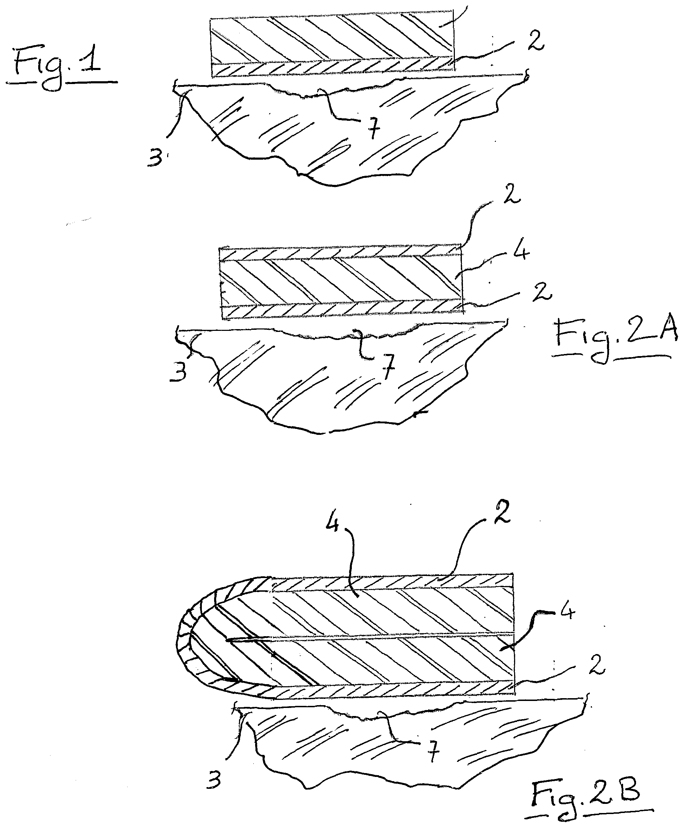

Referring now to schematic Figures 1 to 2B of the drawings, the wound dressing 1 comprises an absorbent component 4, and a wound contacting layer 2 made of nanofibers of fibroin having the above disclosed features. The fibroin layer is in contact with wound 7; in general, a part of the fibroin layer 2, usually the peripheral portion, will contact also the skin 3 of the user.

Similarly Figure 2A shows a wound dressing wherein the absorbent component is sandwiched between two layers of fibroin nanofibers. As it can be seen from Figure 2B, such a device may be obtained by simply folding a device made of one fibroin layer and one absorbent layer; therefore, in this embodiment, the layer of absorbent material is folded so as to provide an absorbing layer actually made of two layers superimposed and connected together at one end.

The wound dressing 1 comprises an absorbent component 4, a wound contacting layer 2 made of nanofibers of fibroin having the above disclosed features.

The device of Figures 2A and 2B is a preferred device, wherein the absorbent component 4 is sandwiched between two layers 2 of fibroin nanofibers, which can be obtained by simply folding a device made of one fibroin layer and one absorbent layer, without sealing the three free edges of the device. When desired of needed, such a device allows a tailored treatment with therapeutic agents; in this case, the healthcare personnel may open, i.e. unfold, the device of Figures 2A and 2B, place the appropriate agent to the absorbent layer (4) and put the edges close again before applying it to the wound of the subject to be treated.

In an exemplary embodiment, the device of fig. 2B is partially unfolded, i.e. opened, and sterile physiological solution is added to the layer 4 of absorbing material. The aim of this wet device is to provide humidity to a wound with low exudate; because the fibroin layer 2 is hydrophobic, water/humidity is slowly permeating through it both towards the wound and partly through the upper, i.e. external layer of fibroin. According to another exemplary embodiment, a medical composition may be added to the absorbent layers 2.

Viceversa, the absorbent material 4 will become humid by absorbing water from the fluids of the wound and thanks to this the absorbent layer will maintain the wound humid enough to help its healing process.

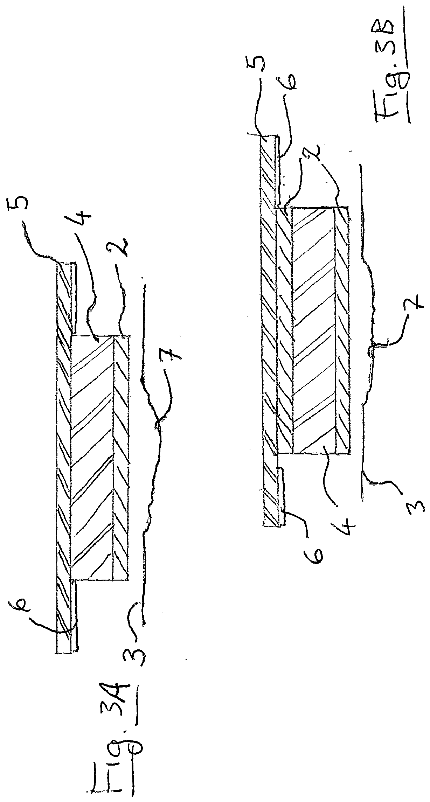

Referring now to Figures 3A and 3B, in the shown exemplary embodiment, the said structure of the Figure 1 and 2 have an additional adhesive layer or other polymer (i.e. silicone or PDMS) layer so that the wound contacting layer 2 forms an island in the adhesive layer or polymer layer 6. The adhesive or other polymer layer 6 is provided on one side of a film backing 5 bonded to the component 2.

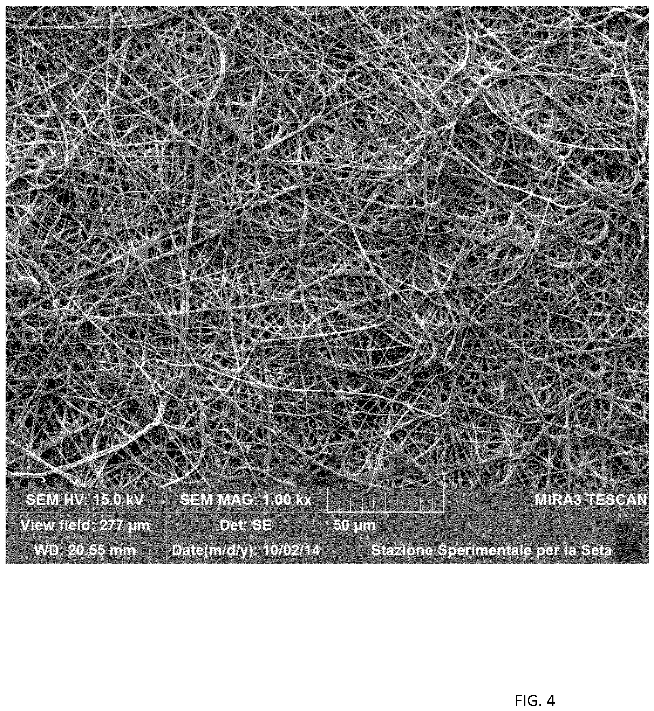

Figure 4 shows a scanning electron microscope image with a 1000x magnification.

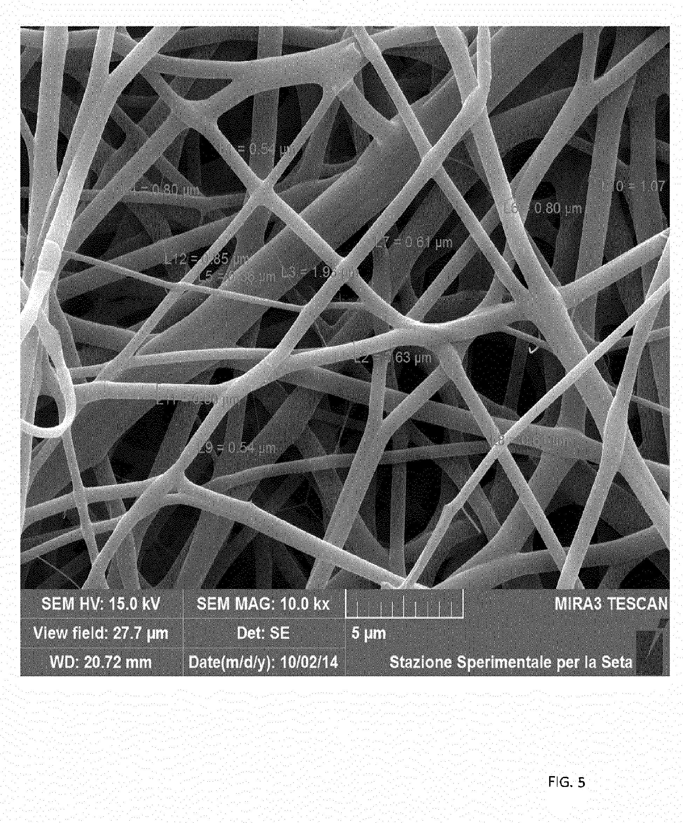

Figure 5 shows a scanning electron microscope image with a 10000x magnification and a measurement analysis of the diameter of the fibers; the pictures of Figures 4 and 5 are taken with scanning electron microscope SEM-FEG MIRA3. The electron microscope measures the diameter with a software that is provided by the manufacturer; the software uses scale bar calibration. It can be appreciated that the average diameter value of the measured fibers of Figure 5 is 780 nm.

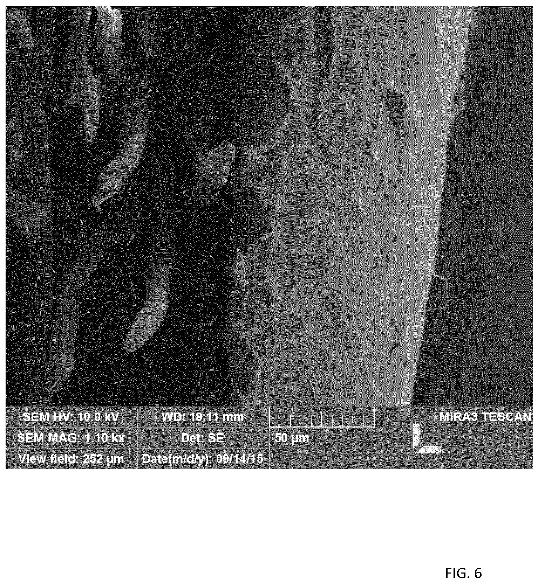

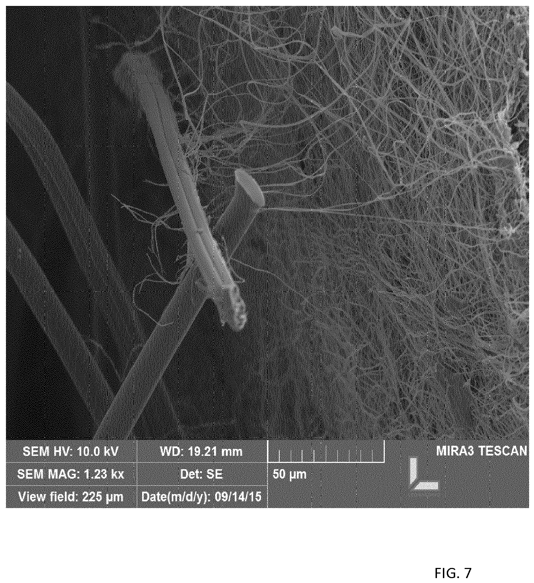

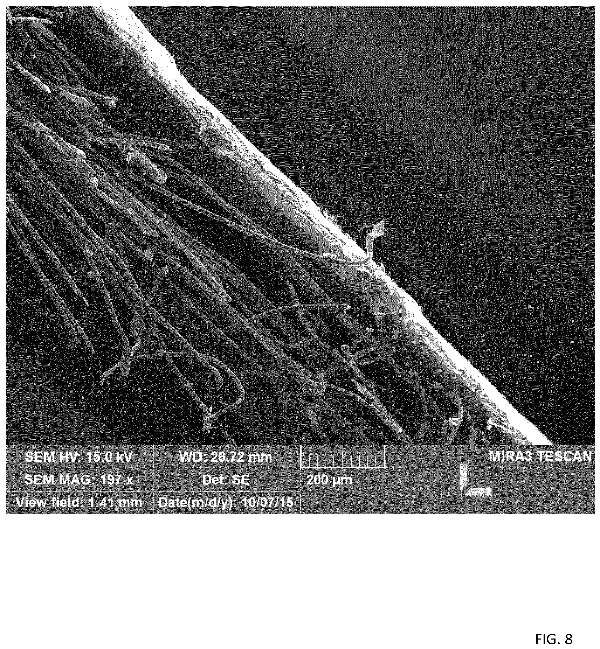

Figures 6, 7 and 8 show scanning electron microscope images of the two layers of a device with different magnification, wherein the difference in the diameter of the fibers (fibroin vs absorbent agent made of viscose/polyester). As it can be appreciated, the fibroin fibers have an average diameter that is less than 800 nm whereas the absorbent fibers have an average diameter higher than 1 or more microns.

As mentioned, the invention provides for the use of nanofibers of silk fibroin to provide the bacteria and microorganisms with a structure where they will adhere and remain entrapped in. Thus, by removing the dressing device of the invention, the bacteria will be removed too.

The film backing and the adhesive are not necessary when the wound device is used as primary medication of a wound, and could be maintained in position on the wound e.g. by means of a traditional medical gauze or bandage or plasters or other similar fixing devices. The adhesive film backing is also not necessary, but could be replaced by another polymer film or layer for example in an embodiment of the invention which could comprise an additional layer suitable to be used together with a negative pressure apparatus.

According to another of its aspects, the invention relates to the use of the device of the invention for negative pressure wound treatment (NPWT).

The wound dressing of the invention can also be used as an absorber for bodily fluids, i.e. as part of or in a diaper for children and adults and in women absorbents. In another exemplary embodiment of the invention, the fibroin nanofibers contain, i.e. a biocidal substance or compound. The biocidal substance or compound is preferably present in the fibroin solution before the solution is electrospun; according to the type of biocidal substance, there may be a release of the biocidal substance from the nanofibers of fibroin. In a preferred embodiment, the biocidal substance is permanently bound to the fibroin; exemplary substances of this type could be permanent antimicrobial substances based on quaternary ammonium compounds.

According to another of its aspects, the invention relates to a method for treating wounds which comprises applying to a subject in need thereof a device according to the invention.

The method of the invention includes negative pressure wound treatment (NPWT).

With reference to Figures 2A and 2B, the method also includes the step of placing a therapeutical agent onto the absorbent (4) and putting the edges close again before applying it to the wound of the subject to be treated.

According to another of its aspects, the invention relates to a process for the preparation of a device according to the invention which comprising the following steps:
a. pure fibroin meshes are solubilized by contact with CaCl₂ and formic acid in mild stirring conditions, at room temperature;
b. the viscous solution obtained in step (a) is poured on a suitable container and left to let formic acid evaporate;
c. the mixture fibroin + CaCl₂ of step (b) is then purified from CaCl₂ to obtain a fibroin film;
d. the film of step (c) is teared off and left to dry;
e. the films are treated with formic acid, under mild stirring conditions and the solution of fibroin and formic acid is transferred into syringes to be electrospun.

Pure fibroin meshes are obtained according to known methods.

According to a preferred embodiment, the absorber layer is selected from viscose fibers, polyester fibers, cellulose fibers, cotton fibers, foams, gel forming particles, gel forming fibers, gauze and wound dressing parts with liquid absorbing properties, preferably a mixture of viscose/polyester in a weight ratio 70/30.

The invention also relates to a wound dressing device obtained or obtainable by the process above.

The preferred embodiments disclosed for the device also applied to the process of the invention.

## Claims

1. A layer of fibroin fibers for a wound dressing device comprising at least one layer (2) of silk fibroin fibers, wherein said fibroin fibers are nanofibers, and said layer of fibroin fibers has a weight in the range of 10 to 40 g/m² preferably 25-35 g/m².

2. The layer according to claim 1, wherein said fibroin fibers layer (2) is made of 100% fibroin.

3. The layer according to claim 1 or 2, wherein said fibroin fibers are free from polyethylene oxide.

4. The wound dressing according to any previous claim, wherein said fibers of said fibroin layer (2) have an average fiber diameter of less than 900 nm, preferably within the range of 200 to 800 nm, more preferably within the range of 400 to 700 nm.

5. The layer according to any previous claim, wherein the fibroin layer (2) is an electrospun fibroin layer.

6. The layer according to any previous claim, wherein said fibroin layer (2) is free from drugs, active ingredients, or biocidal substances.

7. A wound dressing device (1) comprising a layer of fibroin fibers according to any previous claim and including an absorbent component (4).

8. The wound dressing device (1) according to claim 7, wherein the fibers of said fibroin layer (2) have an average diameter within the range of 200 to 800 nm, preferably 400 to 700 nm.

9. The wound dressing device (1) according to claim 8, wherein said absorbent component is positioned between two layers of fibroin.

10. The wound dressing device (1) according to claim 9, which is obtained by folding a device made of one fibroin layer and one absorbent layer.

11. The wound dressing device (1) according to any claim 7 to 10, wherein said absorbent component (4) comprises at least one material selected from viscose fibers, polyester fibers, cellulose fibers, cotton fibers, foams, gel forming particles, gel forming fibers, gauze and wound dressing parts with liquid absorbing properties.

12. The wound dressing device (1) according to claim 11, wherein said absorbent component (4) is a mixture of viscose/polyester in a weight ratio 70/30.

13. A wound dressing device (1) according to any claim 7 to 12, wherein said absorbent component further comprises a biocidal substance.

14. A wound dressing device (1) according to any claim 7 to 13, further comprising means (5, 6) to adhere at least part of said dressing to the skin (3) of a user.

15. A wound dressing device (1) according to any claim 7 to 14, wherein said silk fibroin fibers are free from polyethylene oxide.
